(19) Europäisches Patentamt
European Patent Office
Office européen des brevets

(11) Veröffentlichungsnummer: **0 460 479 A1**

(12) **EUROPÄISCHE PATENTANMELDUNG**

(21) Anmeldenummer: **91108516.5**

(22) Anmeldetag: **25.05.91**

(51) Int. Cl.5: **C07D 285/13**, C07D 285/125, A01N 43/82

(30) Priorität: **08.06.90 DE 4018352**

(43) Veröffentlichungstag der Anmeldung: **11.12.91 Patentblatt 91/50**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Förster, Heinz, Dr.**
**Am Eckbusch 47**
**W-5600 Wuppertal 1(DE)**
Erfinder: **Diehr, Hans-Joachim, Dr.**
**Höhe 35**
**W-5600 Wuppertal 11(DE)**
Erfinder: **Baasner, Bernd, Dr.**
**Wagnerstrasse 83**
**W-5060 Bergisch Gladbach 2(DE)**
Erfinder: **Kysela, Ernst, Dr.**
**Virchowstrasse 14**
**W-5060 Bergisch Gladbach 1(DE)**
Erfinder: **Marhold, Albrecht, Dr.**
**Carl-Duisberg-Strasse 329**
**W-5090 Leverkusen 1(DE)**
Erfinder: **Gassen, Karl-Rudolf, Dr.**
**Auenweg 6a**
**W-5068 Odenthal(DE)**
Erfinder: **Schmidt, Robert R., Dr.**
**Im Waldwinkel 110**
**W-5060 Bergisch Gladbach 2(DE)**

(54) **Cycloalkyl-substituierte Thiadiazolyloxyessigsäureamide.**

(57) Die Erfindung betrifft neue cycloalkyl-substituierte Thiadiazolyloxyessigsäureamide der Formel

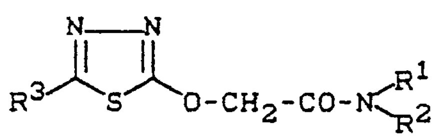

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann, und

$R^3$ für gegebenenfalls substituiertes Cycloalkyl steht,

ferner ein Verfahren und neue Zwischenprodukte zu ihrer Herstellung sowie ihre Verwendung als Herbizide.

EP 0 460 479 A1

Die Erfindung betrifft neue cycloalkyl-substituierte Thiadiazolyloxyessigsäureamide, ein Verfahren und neue Zwischenprodukte zu ihrer Herstellung sowie ihre Verwendung als Herbizide.

Es ist bereits bekannt, daß bestimmte Phenyl-substituierte Thiadiazolyloxyessigsäureamide herbizide Eigenschaften aufweisen (vergleiche DE-OS 30 38 635, EP-A 29 171, EP-A 60 426). Die herbizide Wirksamkeit dieser Verbindungen ist jedoch nicht immer ganz zufriedenstellend.

Es wurden nun neue cycloalkyl-substituierte Thiadiazolyloxyessigsäureamide der allgemeinen Formel (I)

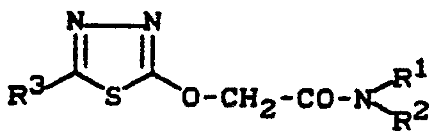

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann, und

$R^3$ für gegebenenfalls substituiertes Cycloalkyl steht,

gefunden.

Weiter wurde gefunden, daß man die neuen cycloalkyl-subsituierten Thiadiazolyloxyessigsäureamide der allgemeinen Formel (I) erhält, wenn man Methylsulfonylthiadiazole der allgemeinen Formel (II)

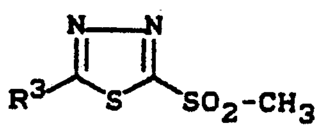

(II)

in welcher

$R^3$ die oben angegebene Bedeutung hat,

mit Hydroxyessigsäureamiden der allgemeinen Formel (III)

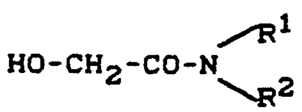

(III)

in welcher

$R^1$ und $R^2$ die oben angegebenen Bedeutungen haben,

gegebenenfalls in Gegenwart eines Verdünnungsmittels,
gegebenenfalls in Gegenwart eines Säurebindemittels und
gegebenenfalls in Gegenwart eines Katalysators umsetzt.

Schließlich wurde gefunden, daß die neuen cycloalkyl-substituierten Thiadiazolyloxyessigsäureamide der allgemeinen Formel (I) interessante herbizide Eigenschaften besitzen.

Gegenstand der Erfindung sind vorzugsweise Verbindungen der Formel (I), in welcher

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl,

welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist, und

$R^3$ für $C_3$-$C_6$-Cycloalkyl steht, welches gegebenenfalls durch Halogen, $C_1$-$C_4$-Alkyl und/oder $C_1$-$C_4$-Halogenalkyl substituiert ist.

Die Erfindung betrifft insbesondere Verbindungen der Formel (I), in welcher

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydrochinolinyl stehen, und

$R^3$ für jeweils durch Fluor und/oder Chlor und gegebenenfalls zusätzlich durch Methyl substituiertes Cyclopropyl oder Cyclobutyl steht.

Besonders bevorzugt sind die Verbindungen der Formel (Ia)

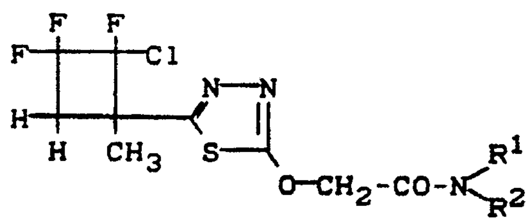

(Ia)

in welcher

$R^1$ und $R^2$ die oben als insbesondere bevorzugt angegebenen Bedeutungen haben.

Verwendet man beispielsweise 2-Methylsulfonyl-5-(1-methyl-2-chlor-2,3,3-trifluor-1-cyclobutyl)-1,3,4-thiadiazol und Hydroxyessigsäurediethylamid als Ausgangsstoffe, so läßt sich der Reaktionsablauf beim erfindungsgemäßen Verfahren durch das folgende Formelschema darstellen:

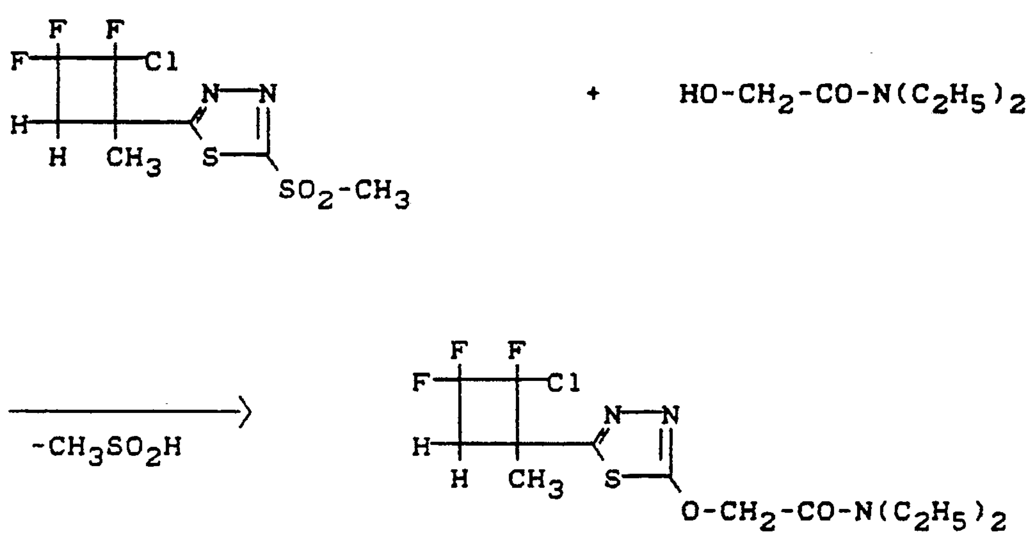

Die beim erfindungsgemäßen Verfahren zur Herstellung von Verbindungen der Formel (I) als Ausgangsstoffe zu verwendenden Methylsulfonylthiadiazole sind durch die Formel (II) allgemein definiert.

In Formel (II) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde.

Die Ausgangsstoffe der Formel (II) sind noch nicht aus der Literatur bekannt und sind ebenfalls Gegenstand der vorliegenden Erfindung.

Man erhält die neuen Verbindungen der Formel (II), wenn man Methylthio-thiadiazole der allgemeinen Formel (IV)

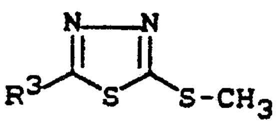

(IV)

in welcher

$R^3$ die oben angegebene Bedeutung hat,

mit einem Oxidationsmittel, wie z. B. Hydrogenperoxid, gegebenenfalls in Gegenwart eines Katalysators, wie z. B. Natriumwolframat, und gegebenenfalls in Gegenwart von Verdünnungsmitteln, wie z.B. Wasser, Ameisensäure und/oder Essigsäure, bei Temperaturen zwischen 0 °C und 100 °C umsetzt.

Die als Zwischenprodukte benötigten Methylthiothiadiazole sind durch die Formel (IV) allgemein definiert. In Formel (IV) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde.

Die Zwischenprodukte der Formel (IV) sind noch nicht aus der Literatur bekannt und sind gleichfalls Gegenstand der vorliegenden Erfindung.

Man erhält die neuen Verbindungen der Formel (IV), wenn man Carbonsäurechloride der allgemeinen Formel (V)

$R^3$-CO-Cl (V)

in welcher

$R^3$ die oben angegebene Bedeutung hat,

mit Dithiocarbazinsäuremethylester in Gegenwart eines Reaktionshilfsmittels, wie z. B. Pyridin, und gegebenenfalls in Gegenwart eines Verdünnungsmittels, wie z. B. Tetrahydrofuran, und anschließend mit Schwefelsäure, jeweils bei Temperaturen zwischen -10 °C und +30 °C umsetzt (vergleiche die Herstellungsbeispiele).

Die Carbonsäurechloride der Formel (V) sind bekannt und/oder können nach an sich bekannten Verfahren hergestellt werden.

Noch nicht aus der Literatur bekannt, aber Gegenstand einer älteren, nicht vorveröffentlichten Patentanmeldung (vergleiche DE-P 39 33 750 / LeA 27 228 vom 10.10.1989) ist 2-Chlor-1-methyl-2,3,3-trifluorcyclobutan-1-carbonsäurechlorid der Formel (Va)

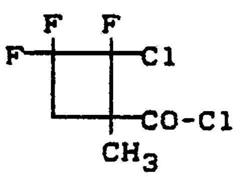

(Va)

Die Herstellung der Verbindung der Formel (Va) ist in den Herstellungsbeispielen beschrieben.

Die beim erfindungsgemäßen Verfahren zur Herstellung von Verbindungen der Formel (I) weiter als Ausgangsstoffe zu verwendenden Hydroxyessigsäureamide sind durch die Formel (III) allgemein definiert.

In Formel (III) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Hydroxyessigsäureamide der Formel (III) sind bekannt und/oder können nach an sich bekannten

Verfahren hergestellt werden (vgl. US-P 4 509 971 und US-P 4 645 525; ferner US-P 4 334 073, DE-OS 3 038 598, DE-OS 3 038 636, EP-A 37 526, EP-A 348 737, DE-OS 38 19 477).

Das erfindungsgemäße Verfahren zur Herstellung der neuen cycloalkyl-substituierten Thiadiazolyloxyessigsäureamide der Formel (I) wird vorzugsweise unter Verwendung von Verdünnungsmitteln duchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether, Diisopropylether, Dibutylether, Diisobutylether, Glycoldimethylether, Tetrahydrofuran und Dioxan, Alkohole, wie z. B. Methanol, Ethanol, Propanol, Isopropanol oder Butanol,

Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamid, Dimethylacetamid und N-Methyl-pyrrolidon, Nitrile, wie z. B. Acetonitril und Propionitril, Sulfoxide, wie z. B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z. B. Natrium-, Kalium-, Magnesium- und Calciumoxid, Hydroxide, wie z. B. Natrium-, Kalium-, Magnesium- und Calciumhydroxid und/oder Carbonate, wie z. B. Natrium-, Kalium-, Magnesium- und Calciumcarbonat verwendet.

Der Zusatz von 0,01 bis 10 Gew.-% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:

Tetrabutylammoniumchlorid, Tetrabutylammoniumbromid, Tributyl-methylphosphoniumbromid, Trimethyl-$C_{13}/C_{15}$-alkyl-ammoniumchlorid, Dibenzyl-dimethyl-ammonium-methylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +100 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol Methylsulfonylthiadiazol der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-,

Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur Bekämpfung von monokotylen Unkräutern in dikotylen Kulturen, zum Teil auch in monokotylen Kulturen (wie z. B. in Reis). Sie sind vor allem zur Bekämpfung von Schadgräsern (wie z. B. Echinochloa) in Wasserreis geeignet.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen

appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 60 und 4000 g Wirkstoff pro Hektar Bodenfläche, vorzugsweise zwischen 120 und 2000 g pro ha.

Die Herstellung und die Verwendung der erfindungsgemäßen Wirkstoffe geht aus den nachfolgenden Beispielen hervor.

Herstellungsbeispiele:

Beispiel 1

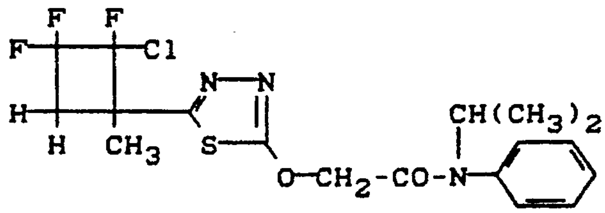

Eine Lösung von 0,92 g (23 mMol) Natriumhydroxid in 6 ml Wasser wird unter Rühren zu einer auf -20 °C abgekühlten Mischung aus 7,6 g (23 mMol) 2-Methylsulfonyl-5-(1-methyl-2-chlor-2,3,3-trifluor-1-cyclobutyl)-1,3,4-thiadiazol, 4,4 g (23 mMol) Hydroxyessigsäure-N-isopropyl-anilid und 50 ml Aceton tropfenweise gegeben und die Reaktionsmischung wird 15 Stunden unter Kühlen mit einer Eis-Kochsalz-Mischung gerührt. Anschließend wird mit Essigsäure angesäuert und im Wasserstrahlvakuum eingeengt. Der Rückstand wird mit Chloroform/Wasser geschüttelt, die organische Phase abgetrennt, mit Natriumsulfat getrocknet und filtriert. Vom Filtrat wird das Lösungsmittel im Wasserstrahlvakuum sorgfältig abdestilliert.

Man erhält 9,3 g (94 % der Theorie) 5-(1-Methyl-2-chlor-2,3,3-trifluor-1-cyclobutyl)-1,3,4-thiadiazol-2-yl-oxyessigsäure-N-isopropyl-anilid als öligen Rückstand vom Brechungsindex $n_D^{20}$ = 1,5169.

Analog Beispiel 1 und entsprechend der allgemeinen Beschreibung des erfindungsgemäßen Herstellungsverfahrens können beispielsweise auch die in der nachstehenden Tabelle 1 aufgeführten Verbindungen der Formel (I) bzw. (Ia) hergestellt werden.

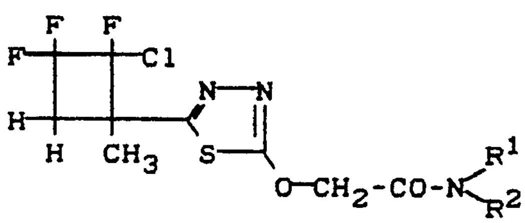

(Ia)

Tabelle 1: Beispiele für die Verbindungen der Formel (Ia)

| Beisp. Nr. | $R^1$ | $R^2$ | physikal. Daten |
|---|---|---|---|
| 2 | $-CH_3$ | $-CHC_2H_5$<br>\|<br>$CH_3$ | $n_D^{20}$ = 1,4839 |
| 3 | $-CH_3$ | 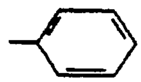 | $n_D^{20}$ = 1,5285 |
| 4 | $-C_2H_5$ | 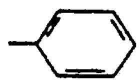 | |
| 5 | $-CH(CH_3)_2$ | 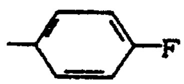 | |
| 6 | $-CH(CH_3)_2$ | $-OCH_2CH_2OC_2H_5$ | |
| 7 | $-CH_2-CH=CH_2$ | $-CH_2-CH=CH_2$ | |
| 8 | $-CH(CH_3)_2$ | 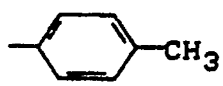 | |

**Tabelle 1:** (Fortsetzung) Beispiele für die Verbindungen der Formel (Ia)

| Beisp. Nr. | $R^1$ | $R^2$ | physikal. Daten |
|---|---|---|---|
| 9 | $-CH(CH_3)_2$ | 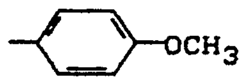 | |
| 10 | $-CH(CH_3)_2$ | 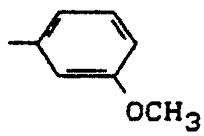 | |
| 11 | $-CH(CH_3)_2$ | 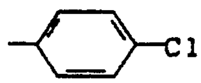 | |
| 12 | $-C_2H_5$ | 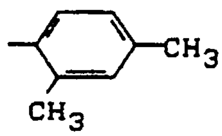 | |
| 13 | $-CH_3$ | 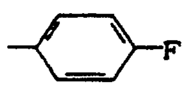 | |
| 14 | $-CH_3$ | 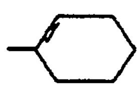 | |
| 15 | $-(CH_2)_6-$ | | |
| 16 | 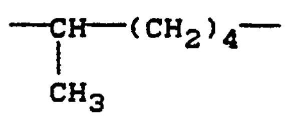 | | |

Ausgangsstoffe der Formel (II) und (IV)

Beispiel (II-1) / (IV-1)

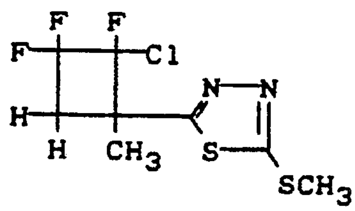

(IV-1)

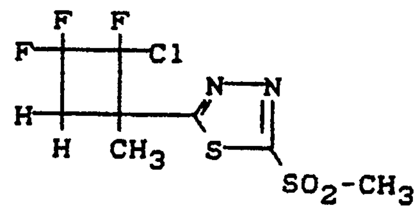

(II-1)

22,1 g (0,10 Mol) 1-Methyl-2-chlor-2,3,3-trifluor-cyclobutan-1-carbonsäurechlorid werden tropfenweise zu einer auf -5° C abgekühlten Mischung aus 12,2 g (0,10 Mol) Dithiocarbazinsäuremethylester,9,0 g (0,11 Mol) Pyridin und 100 ml Tetrahydrofuran gegeben und das Gemisch wird nach Entfernen des Kühlbades 2 Stunden gerührt. Unter Eiskühlung werden dann 50 ml konzentrierte Schwefelsäure dazu gegeben und das Reaktionsgemisch wird 15 Stunden bei 20° C gerührt. Anschließend wird mit Eiswasser verdünnt und mit Chloroform geschüttelt. Die organische Phase wird abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 29 g (ca. 100 % der Theorie) 2-Methylthio-5-(1-methyl-2-chlor-2,3,3-trifluor-1-cyclobutyl)-1,3,4-thiadiazol als öligen Rückstand. Dieses Produkt (29 g) wird in 80 ml Essigsäure aufgenommen und nach Zugabe von 0,5 g Natriumwolframat werden 25 ml einer 30%igen wässrigen Hydrogenperoxid-Lösung tropfenweise dazugegeben. Das Reaktionsgemisch wird 60 Minuten bei 40° C bis 50° C gerührt und anschließend mit Eiswasser verdünnt. Das hierbei abgeschiedene ölige Produkt wird in Chloroform aufgenommen, diese Lösung mit Wasser und 5%iger Natriumbicarbonatlösung gewaschen, mit Natriumsulfat getrocknet und filtriert. Vom Filtrat wird das Lösungsmittel im Wasserstrahlvakuum abdestilliert und der feste Rückstand aus Isopropanol umkristallisiert.

Man erhält 13,1 g (41 % der Theorie) 2-Methylsulfonyl-5-(1-methyl-2-chlor-2,3,3-trifluor-1-cyclobutyl)-1,3,4-thiadiazol vom Schmelzpunkt 63° C.

Ausgangsverbindung der Formel (Va)

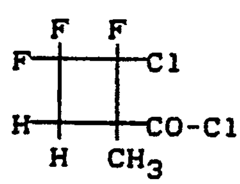

1. Stufe

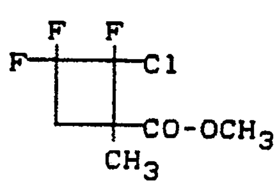

750 g (7,5 Mol) Methacrylsäuremethylester, 700 g (6,0 Mol) Chlortrifluorethen und 3 g Hydrochinon werden in einem Stahlautoklaven 12 Stunden auf 120° C erhitzt. Das Produkt wird direkt fraktioniert.

Man erhält 780 g (60 % der Theorie) 1-Methyl-2-chlor-2,3,3-trifluor-cyclobutan-1-carbonsäure-methylester, Siedepunkt: 57-59° C/18,6 mbar.

2. Stufe

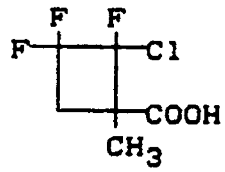

573 g (2,65 Mol) 1-Methyl-2-chlor-2,3,3-trifluor-cyclobutan-1-carbonsäuremethylester, 233 g (5,8 Mol) Natriumhydroxid und 1000 ml Wasser werden drei Stunden bei 80° C gerührt. Es wird mit konz. Salzsäure angesäuert und mit Dichlormethan extrahiert, die organischen Phasen werden getrocknet und destilliert. Man erhält 472 g (88 % der Theorie) 1-Methyl-2-chlor-2,3,3-trifluor-cyclobutan-1-carbonsäure, Siedepunkt: 112-116° C/21,4 mbar (60 % trans-Isomeres, 40 % cis-Isomeres).

3.Stufe

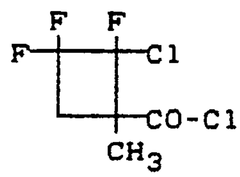

220 g (1,09 Mol) 1-Methyl-2-chlor-2,3,3-trifluor-cyclobutan-1-carbonsäure werden mit 250 g (1,23 Mol) Phthalsäuredichlorid über Nacht bei Raumtemperatur gerührt. Anschließend wird das Säurechlorid abdestilliert.

Man erhält 238 g (99 % der Theorie) 1-Methyl-2-chlor-2,3,3-trifluor-cyclobutan-1-carbonsäurechlorid, Siedepunkt: 54-56° C/30,6 mbar.

Anwendungsbeispiele:

Beispiel A

Test an verpflanztem Wasserreis

Lösungsmittel: 5 Gewichtsteile Aceton
Emulgator : 1 Gewichtsteil Benzyloxypolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat auf die gewünschte Konzentration.

Pflanzgefäße (Oberfläche 1/5000 Ar) werden mit Boden aus einem Reisfeld gefüllt. Zwei Reispflanzen (Sorte: Kinmaze) werden im 2-3 Blattstadium (ca. 10 cm hoch) in die Gefäße verpflanzt. Samen von Echinochloa crus galli werden in die feucht gehaltene Erde ausgesät. 5 Tage nach dem Verpflanzen des Reises wird der Boden bis zu einer Wassertiefe von 3 cm überstaut. Die Wirkstoffzubereitung wird auf die Wasseroberfläche ausgebracht. Die Wirkstoffkonzentration in der Zubereitung spielt keine Rolle, entscheidend ist nur die Aufwandmenge des Wirkstoffs pro Flächeneinheit.

Nach der Anwendung des Wirkstoffs wird für 2 Tage durch die Pflanzgefäße ein vertikal absteigender Wasserstrom mit einer Geschwindigkeit von 2-3 cm pro Tag eingestellt. Danach werden die Testansätze unter Überflutungsbedingungen gehalten, wobei die Wassertiefe 3 cm beträgt.

Nach 4 Wochen wird der Schädigungsgrad der Pflanzen bonitiert in % Schädigung (bzw. Unkrautwirkung) im Vergleich zu einer unbehandelten Kontrolle.

Es bedeuten:

0 % = keine Wirkung (wie unbehandelte Kontrolle)
100 % = totale Vernichtung

In diesem Test zeigt sich die hervorragende Verträglichkeit der erfindungsgemäßen Wirkstoffe - insbesondere der Verbindungen aus den Beispielen 1 und 3 - in Reis bei gleichzeitig sehr guter

Unkrautwirkung.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei halt man die Wassermenge pro Flacheneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Auch in diesem Test zeigen die erfindungsgemäßen Verbindungen der Formel (I) sehr gute Wirksamkeit.

**Patentansprüche**

**1.** Cycloalkyl-substituierte Thiadiazolyloxyessigsäureamide der allgemeinen Formel (I),

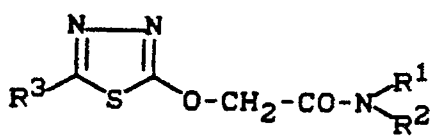

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann, und

$R^3$ für gegebenenfalls substituiertes Cycloalkyl steht.

**2.** Verbindungen der allgemeinen Formel (I) gemäß Anspruch 1, dadurch gekennzeichnet, daß darin

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom, an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanel-

liert ist, und

$R^3$ für $C_3$-$C_6$-Cycloalkyl steht, welches gegebenenfalls durch Halogen, $C_1$-$C_4$-Alkyl und/oder $C_1$-$C_4$-Halogenalkyl substituiert ist.

**3.** Verbindungen der allgemeinen Formel (I) gemäß Anspruch 1, dadurch gekennzeichnet, daß darin

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydrochinolinyl stehen, und

$R^3$ für jeweils durch Fluor und/oder Chlor und gegebenenfalls zusätzlich durch Methyl substituiertes Cyclopropyl oder Cyclobutyl steht.

**4.** Verbindungen der Formel (Ia),

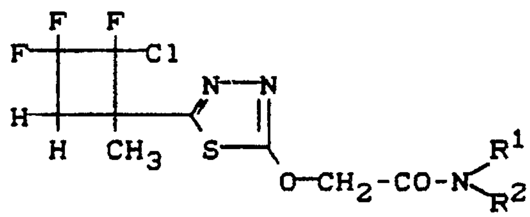

(Ia)

in welcher

$R^1$ und $R^2$ die in Anspruch 3 angegebenen Bedeutungen haben.

**5.** Verfahren zur Herstellung von cycloalkyl-substituierten Thiadiazolyloxyessigsäureamiden der allgemeinen Formel (I) gemäß Anspruch 1, dadurch gekennzeichnet, daß man Methylsulfonylthiadiazole der allgemeinen Formel (II)

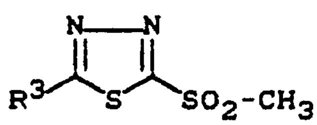

(II)

in welcher

$R^3$ die in Anspruch 1 angegebene Bedeutung hat,

mit Hydroxyessigsäureamiden der allgemeinen Formel (III)

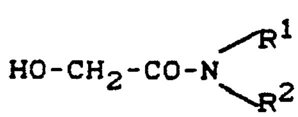

(III)

in welcher

$R^1$ und $R^2$ die in Anspruch 1 angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

**6.** Herbizide Mittel, gekennzeichnet durch einen Gehalt an mindestens einer Verbindung der Formel (I) gemäß Anspruch 1.

7. Verfahren zur Bekämpfung von Unkraut, dadurch gekennzeichnet, daß man eine Verbindung der Formel (I) gemäß Anspruch 1 auf die Unkräuter oder ihren Lebensraum einwirken läßt.

8. Verwendung von Verbindungen der Formel (I) gemäß Anspruch 1 zur Bekämpfung von unerwünschtem Pflanzenwachstum.

9. Verfahren zur Herstellung von herbiziden Mitteln, dadurch gekennzeichnet, daß man Verbindungen der Formel (I) gemäß Anspruch 1 mit Streckmitteln und/oder oberflächenaktiven Mitteln vermischt.

10. Methylthio- und Methylsulfonyl-thiadiazole der Formeln (IV) bzw. (II)

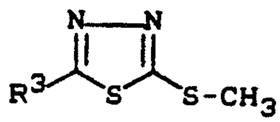

(IV)

bzw.

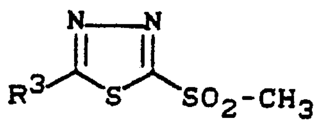

(II)

in welchen
$R^3$ für gegebenenfalls substituiertes Cycloalkyl steht.

Europäisches Patentamt

# EUROPÄISCHER RECHERCHENBERICHT

Nummer der Anmeldung

EP 91108516.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP - A1 - 0 348 736 (BAYER AG) * Ansprüche 1,4,6,7 * -- | 1,2, 4-10 | C 07 D 285/13<br>C 07 D 285/125<br>A 01 N 43/82 |
| P,A | EP - A1 - 0 410 551 (SCHERING AKTIENGESELLSCHAFT) * Anspruch 1 * -- | 10 | |
| A | DE - A1 - 3 722 320 (BAYER) AG) * Anspruch 1 * ---- | 10 | |
| | | | RECHERCHIERTE SACHGEBIETE (Int. Cl.5) |
| | | | C 07 D 285/00 |

Der vorliegende Recherchenbericht wurde für alle Patentansprüche erstellt.

| Recherchenort | Abschlußdatum der Recherche | Prüfer |
|---|---|---|
| WIEN | 02-07-1991 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument